# EUROPEAN PATENT SPECIFICATION

(11) **EP 0 993 617 B1**
(45) Date of publication and mention of the grant of the patent: **04.02.2004**
(21) Application number: 98930954.7
(22) Date of filing: 26.06.1998
(51) Int. Cl.: G01N 33/68, G01N 33/98

(54) **ASSAY FOR CARBOHYDRATE-FREE TRANSFERRIN**
TEST FÜR KOHLENHYDRAT-FREIES TRANSFERRIN
DOSAGE DE TRANSFERRINE EXEMPTE D'HYDRATES DE CARBONE

(30) Priority: 26.06.1997 GB 9713559
(43) Date of publication of application: 19.04.2000
(73) Proprietor: Axis Shield ASA, 0510 Oslo (NO)
(72) Inventor: SUNDREHAGEN, Erling, Axis Biochemicals asa, N-0510 Oslo (NO)
(74) Representative: Dzieglewska, Hanna Eva
(86) International application number: PCT/GB1998/001875
(87) International publication number: WO 1999/000672

(56) References cited:
- EP-A- 0 043 359
- EP-A- 0 324 969
- EP-A- 0 524 583
- WO-A-93/06133
- DE-A- 19 543 569
- US-A- 5 702 904
- PATENT ABSTRACTS OF JAPAN vol. 012, no. 107 (P-686), 7 April 1988 & JP 62 239056 A (WAKO PURE CHEM IND LTD), 19 October 1987
- PEKELHARING J M: "AN ELISA TECHNIQUE FOR MEASUREMENT OF TRANSFERRIN GLYCO-VARIANTS IN BODY FLUIDS USING LECTINS BOUND TO A SOLID PHASE" PROTIDES OF THE BIOLOGICAL FLUIDS,1983, pages 115-118, XP000646760
- PEKELHARING J M ET AL: "LECTIN-ENZYME IMMUNOASSAY OF TRANSFERRIN SIALOVARIANTS USING IMMOBILIZED ANTITRANSFERRIN AND ENZYME-LABELED GALACTOSE-BINDING LECTIN FROM RICINUS COMMUNIS" ANALYTICAL BIOCHEMISTRY, vol. 165, no. 2, 1987, pages 320-326, XP000646761

## Description

This invention relates to an assay method for assessing carbohydrate free transferrin (CFT) for the diagnosis and monitoring of alcoholism, and to kits and apparatus for performing the assay.

Many biological proteins exist in two or more variant forms, frequently differing in the extent of glycosylation of the protein or in the carbohydrate composition *per se*. The relative concentrations of such variants in a given body tissue or fluid are generally constant, but may be disturbed in certain diseases or pathological states, or as a result of other disturbances to the body. The ratio, for example, of glycosylated to non-glycosylated haemoglobins is known to increase in the serum of patents suffering from diabetes. Similarly, some structural proteins for example, myoglobins, may have slight structural differences in different organs and may be released into the bloodstream following cell damage resulting from disease or injury.

Thus, by measuring the levels of the different variants of a protein in the blood or body fluid of interest, a diagnosis or assessment of a disease or cellular damage can be made.

Serum transferrin is a glycoprotein with a molecular weight of about 80 kD which comprises a single polypeptide chain with two N-linked polysaccharide chains. These polysaccharide chains are branched and each chain may terminate in either two or three antennae, each with terminal sialic acid residues.

Wong and Regoeczi, in Int. J. Peptide Res. (1977) 9:241-248, reported that human transferrin was naturally heterogeneous, occurring in variant forms with different levels of sialylation. Until recently, there were generally believed to be six such variants, the pentasialo, tetrasialo, trisialo, disialo, monosialo and asialo transferrins. The existence of the monosialo form is now disputed by some researchers.

The asialo, monosialo, disialo and trisialo variants are often referred to collectively within the field as carbohydrate-deficient transferrin or CDT.

In the normal healthy individual, the tetrasialo variant appears to predominate; however it has been reported that the asialo, monosialo, disialo and, to some degree the trisialo variants, ie. CDT, occur in elevated levels in the blood of alcoholics (see van Eijk *et al*. (1983) Clin Chim Acta 132:167-171, Stibler (1991)Chin Chim 37:2029-2037 and Stibler *et al*. in "Carbohydrate-deficient transferrin (CDT) in serum as a marker of high alcohol consumption", Advances in the Biosciences, (Ed Nordmann *et al*), Pergamon, 1988, Vol. 71, pages 353-357).

CDT has been shown to be an effective marker for - alcohol consumption, in particular for detecting and monitoring chronic alcohol consumption. Monitoring of blood alcohol level is reliable only when blood is sampled within 24 hours of alcohol consumption and conventional tests (for example, quantitation of γ-glutamyltransferase or measurement of mean corpuscular volume) cannot reliably be used to screen for heavy alcohol intake in patients with liver disease.

Early investigations showed that loss of the sialic acid residues correlated with changes in the isoelectric point (pI) of the transferrin molecules, for example, asialotransferrin exhibits a pI of 5.9, disialotransferrin exhibits a pI of 5.7 and so on. Recognition of the fact that the CDT profile of alcohol abusers differs from that of abstainers or normal users, combined with the identification of the relative amounts of each CDT isoform on the basis of pI, has led to the development of several diagnostic assays for CDT which are described in the patent and scientific literature.

In US-A-4626355 (Joustra), Pharmacia AB disclose a chromatographic assay in which a dilute serum sample is passed over an anionic ion exchange column with the pH and ion content of column and sample balanced to permit asialo, monosialo and disialo CDTs to be eluted in an isocratic procedure, while the trisialo and the "normal" tetra and pentasialo variants are retained on the column. The CDT content of the eluate is then determined by competitive immobilization of CDT and radiolabelled-transferrin on an antibody carrying solid phase. In a later modification of this procedure, CDT isoforms of a pI greater than 5.7 are collected and quantified.

In a poster entitled "Rate nephelemetric determination of carbohydrate-deficient transferrin", Schellenberg, Martin, Bénard, Circaud and Weill of Laboratoire de Biochimie CHU Trouseau, Tours, Centre Louis Sevestre, La Membrolle sur Choisille and Beckman France, Gagny, France, described a similar isocratic chromatographic separation in which dilute serum is passed through an anionic ion exchange column, causing the normal transferrin variants to be retained and allowing CDT to elute through. The eluate is then mixed with polyethyleneglycol and centrifuged, an anti-transferrin antibody is added to the supernatant and the CDT content is assessed by nephelometry.

Heil *et al*. (1994) Anaesthetist 43:447-453 have reported a further isocratic chromatographic procedure for CDT determination. In their procedure, a dilute serum sample is passed through an anionic ion exchange resin, again causing normal transferrin variants to be retained, while permitting transit of CDT. The CDT content of the eluate is determined by latex particle enhancement of CDT concentration in an immunoturbidimetric assay procedure.

A further detection system for CDT was disclosed by AXIS Research AS in WO91/19983 (Sundrehagen) wherein labelled antibodies reactive with all transferrin variants, were bound to the variants forming antibody-analyte complexes. These complexes were subjected to fractionation on the basis of differences in charge or pI, for example by isoelectric or by chromato-focusing, and then the amount of label in each fraction was quantified, for example, by fluorescence measurement. This assay relies upon the elution rates being different for the different variant:labelled antibody complexes.

In WO96/26444 (Sundrehagen), AXIS Biochemicals AS disclose yet another method for assessing CDT based on the differences in pI which exist between the different variants. In this method, transferrin containing samples are contacted with an anion ion exchange resin at a pH such as to cause all the CDT variants to be retained. An eluant is then applied to elute the CDT from the column. The CDT collected in this way is substantially free from "normal" tetra and pentasialo transferrin. The CDT collected is then analysed and the variants therein are quantified.

It has recently been reported by Dumon *et al*. (1996) Clin. Biochem. 29(6): 549-553, that of the different CDT variants, the most informative from a diagnostic and assessment point of view, is disialo transferrin and an assay based on isoelectric focussing and immunofixation of disialotransferrin is proposed.

All such prior art methods for CDT analysis rely on differences in the pI or charge of the different transferrin isoforms. Whilst such assays have found utility and indeed some commercial success, in determination of alcohol consumption, they tend to rely upon relatively complex procedures which are not directly applicable to many of the automated multi-task diagnostic machines commonly used by diagnostic laboratories, or else they may be time consuming or costly to perform.

In particular, the pI or charge based methods of the prior art are primarily centred on procedures involving ion exchange chromatography. The difference in pI between the different transferrin variants is very narrow, down to 1/10 of a pH unit and therefore to effect separation of CDT variants, a very good separation is required. In the case of ion exchange chromatography, this constraint effectively means that a column format must be used; batch filtration-based ion exchange procedures do not provide a sufficient separation or resolution. Column formats are however less preferred in clinical chemistry or diagnostic procedures, due to their time consuming and labour intensive operation, problems of storage and transport, incompatibility with commonly-used systems etc.

There is therefore a continuing need for a CDT assay which is robust, simple and quick to perform and readily amenable to automation or compatible with existing routine clinical diagnostic laboratory procedures. The present invention seeks to address this need.

Traditionally, it has been thought that CDT arises from a loss of the terminal sialic acid residues of the carbohydrate side chains and it is upon this that the various prior art pI or charge based assays have been predicated (namely, that a loss of a charged sugar moiety would alter the charge and pH of the isoform as a whole).

However, recent studies (for example by Landberg *et al*. (1995) Biochem. Biophys. Res. Comm. 210(2): 267-274), have shown, by releasing the N-glycans from each isoform of transferrin and analysing them by high-pH anion exchange chromatography, that contrary to this understanding, the existence of disialo and asialotransferrins appears rather to be correlated with the loss of one or both of the entire carbohydrate chains respectively from the transferrin polypeptide. This "deglycosylation" is not yet fully understood.

The carbohydrate chains may be bi or triantennary and hence each carbohydrate chain in its normal state will carry two or three sialic acid residues, one at the terminus of each antenna. It may be that the carbohydrate chains are cleaved from the transferrin molecules at their base in a single step process, i.e. at an asparagine molecule in the amino-acid backbone of the protein, leaving no sugar residues at that particular glycosylation site. Alternatively, individual or multiple sugar residues may be sequentially lost from transferrin molecules resulting in a gradual loss of carbohydrate content. It is also possible that the CDT transferrin molecules are never properly glycosylated in the first place due to aberrant enzymatic glycosylation processes.

To date, the prior art has favoured the idea that either measurement of all of the CDT variants ie. asialo, monosialo, disialo and trisialo transferrin, or at least two or more CDT variants was necessary to make a meaningful clinical evaluation, or that measurement of the disialotransferrin on its own was necessary.

The recent patent application WO95/04932 of Biolin Medical identified the asialo, monosialo and disialotransferrins collectively, as markers for alcoholism and Heggli *et al*. (1996) Alcohol and Alcoholism 31: 381-384 found that by including trisialotransferrin in the measurements of % CDT, the accuracy with which chronically elevated alcohol intake could be determined was increased.

We have now found that the presence of transferrin isoforms which are completely devoid of carbohydrate ie. carbohydrate free transferrin CFT is a strong indicator of alcoholism in the absence of any knowledge of the prevalence of any other CDT variants (ie. monosialo, disialo or trisialotransferrin variants).

It has surprisingly been shown that measurement of all CDT variants ie. asialo, monosialo, disialo and trisialo-transferrin, is unnecessary for a clinically valuable assessment of alcoholism and that determination of carbohydrate-free transferrin or CFT is sufficient.

Thus, according to one aspect, the present invention provides a method for the determination of carbohydrate-free transferrin in a body fluid for use in the assessment of alcohol consumption, said method comprising
(a) contacting a sample of said body fluid with a carbohydrate-binding ligand, to bind any carbohydrate or carbohydrate-containing moieties in said sample to said ligand;
(b) separating a fraction not binding to said ligand and
(c) determining the content of transferrin in said fraction.

By "carbohydrate-free" is meant any transferrin molecule which has lost both of its carbohydrate side chains and is substantially free of any residual N-linked oligosaccharide moieties. Substantial absence of carbohydrate may be determined *inter alia* by lack of any detectable binding to lectins, or other carbohydrate binding proteins, for example, to RCA-I (Ricinus communis agglutinin) or combinations of RCA-I with sialic acid binding lectins. Generally speaking, transferrin preparations in which at least 60% or, more preferably, at least 70 or 80% of the transferrin molecules do not carry a carbohydrate chain or a residue thereof may be regarded as CFT. A CFT preparation may for example comprise 90 or 95% transferrin molecules which do not carry a carbohydrate chain or a' residue thereof.

The body fluid used in the assay method of the invention may be any transferrin-containing body fluid for example, synovial fluid, amniotic fluid or cerebrospinal fluid, but will generally be blood or a blood derived sample. When this is the case, the sample used for analysis will preferably be cell-free and hence, either serum or plasma may be used. The sample may be treated prior to being used in the assay method of the invention, for example, it may be diluted by adding a buffer or other aqueous medium.

In carrying out the method of the invention, the sample is essentially separated into one or more fractions which bind to the carbohydrate-binding ligand and a fraction which does not. This "non-binding" fraction may thus be regarded as substantially free of carbohydrate (ie. at least 60% of the transferrin molecules being free of carbohydrate, eg. at least 70, 80, 90 or 95% being free of carbohydrate).

In this regard, it will be understood by the skilled reader that the nature of scientific and analytical laboratory procedures and biological material is such that absolute precision and uniformity of behaviour can never be guaranteed and that 100% separation may not always be achieved. In any such system some tolerance must be allowed for and this is a principle accepted in the art. In the separation system of the present invention clinical utility may be preserved even though separation may not be 100% complete.

Any transferrin contained in this substantially carbohydrate-free fraction will thus be CFT, and an assessment or determination of the transferrin content of this fraction will provide an assessment or determination of the CFT content of the sample.

The assay method of the invention thus provides a convenient method for the determination of alcohol consumption by assaying CFT in a body fluid, preferably a blood-derived body fluid, and may particularly find utility in the diagnosis and monitoring of alcoholism or alcohol abuse.

As mentioned above, CFT has been shown according to the present invention, to be a good indicator or marker for alcoholism or alcohol abuse, and by assaying the CFT content in samples of body fluid, a distinction may be found between alcoholics and alcohol abusers and non-alcohol abusers or social drinkers.

As used herein, the terms "determining" or "assessing" include both quantitation in the sense of obtaining an absolute value for the amount or concentration of CFT in the sample, and also semiquantitative and qualitative assessments or determinations. An index, ratio, percentage or similar indication of the level or amount of CFT, for example relative to total transferrin (ie. all transferrin variants) may be obtained.

The amount of CFT may be determined directly by measuring the transferrin not bound by the carbohydrate binding ligand ie. by determining the transferrin content in the "carbohydrate free" "non-binding" fraction which is separated. Alternatively it may be determined indirectly by determining the amount of transferrin bound to a carbohydrate binding ligand and subtracting this from the total amount of transferrin present in the sample. Generally, the direct approach is preferred.

Any carbohydrate-binding ligand or any combination thereof may be used to separate the CFT from other transferrin variants. This includes any ligand capable of binding to any carbohydrate or oligosaccharide or sugar structures. One or more carbohydrate-binding ligands may be used in the method of the invention. Generally, the carbohydrate-binding ligand will be a protein, and very many such carbohydrate-binding proteins are known in the art and are widely described in the literature. The carbohydrate-binding protein may, for example, be an antibody, either polyclonal or monoclonal, or may be an antibody fragment for example F(ab), F(ab')₂ or F(v) fragments. The antibodies or antibody fragments may be monovalent or divalent and they may be produced by hybridoma technology or be of synthetic origin, via recombinant DNA technology or chemical synthesis. Single chain antibodies could for example be used. The antibody may be directed or raised against any of the carbohydrate components or structures making up the carbohydrate chains of glycosylated transferrin variants. Thus, for example, an antibody reactive with or selective for sialic acid residues might be used. Such an antibody is used in the Sialic Acid Deficient Enzyme Immunoassay (SDT-EIA) available from Medichem, Stuttgart, Germany and described in WO97/19355.

More preferably, the carbohydrate-binding protein may be a lectin, used singularly or in combination with other lectins or with other types of carbohydrate-binding proteins, for example, antibodies. Any lectin known in the art may be used in the assay method of the invention and it may be of plant, animal, microbiological or any other origin. The literature is replete with references to different lectins which might be used, and many may be obtained commercially, for example, from Sigma.

Thus, included within the general term "lectin" as used herein, in addition to the classical plant lectins such as Concanavalin A (Con A), are carbohydrate binding proteins from microorganisms (for example, viral haemagglutinins) and higher organisms, including for example, invertebrates and mammals. Such mammalian carbohydrate binding proteins include selectins and other mammalian lectins or cell adhesion molecules (see for example Varki (1992) Current Opinion in Cell Biology 4:257-266).

The functional requirement of the carbohydrate binding ligands, rendering them suitable for use in the assay method of the present invention, is that they be capable of separating CFT from other transferrin variants bearing one or both oligosaccharide chains, in an entire or degraded form.

Whilst a single type of carbohydrate-binding ligand may be used according to the invention, conveniently more than one such binding ligand may be used and even more conveniently, a number of different carbohydrate binding ligands, each with differing sugar or oligosaccharide binding capacities. Thus, in one preferred embodiment, a panel of different ligands with differing selectivity and specificity is used.

Combinations of different carbohydrate ligands are preferred due to the increased binding capacity which may be provided by two or more ligands and hence better separation of the transferrin isoforms. Many carbohydrate binding ligands for example, lectins, have low binding affinities for their sugar or oligosaccharide binding partners and the synergistic binding capacity provided by more than one ligand is advantageous.

Examples of suitable lectins are RCA-I (*Ricinus communis agglutinin*) which binds terminal galactose (Kornfeld *et al*. (1981) J. Biol. Chem. **256:**6633) or Con-A (Concanavalin A), which is known to bind asparagine-linked oligosaccharides high in mannose. Other possibilities are *Crotalaria juncea* lectin which binds galactose residues (Ersson (1977) Biochim. Biophys. Acta **494:**51-60), Wheatgerm agglutinin or *Limulus polyphenus* lectin which bind sialic acid (Mandal and Mandal (1990) Experientia **46:**433-441) or *Sambucus nigra agglutinin L* which binds Neu5Ac/(∝2-6)Gal/GalNAc (Shibuya *et al*. (1987) J. Biol. Chem. **262:**1596). As an example of a lectin derived from a micro-organism, a sialic acid specific lectin has recently been purified from the gut dwelling organism *Helicobacter pylori* (Lelwala-Guruge *et al*. (1993) APMIS 101:695-702).

Lectins of varying selectivity and specificity are known. Whereas some lectins may bind to a single sugar residue in a particular location on an oligosaccharide chain, for example RCA-I (from *Ricinus communis*) binds only to terminal galactose residues, some may bind to complex oligosaccharide determinants for example *Sambucus nigra L* which binds Neu5Ac/(∝2-6)Gal/GalNAc. All are within the scope of the present invention.

Sialic acid binding lectins and other proteins represent a class of carbohydrate binding proteins of particular utility in the present invention (see the following, for example, for lists of suitable lectins and their sources: Mandal and Mandal(1990) Experientia 46:433-441); Zeng (1992) Z. Naturforsch, 47c:641-653 and Reuter and Schauer in Methods in Enzymology, Vol. 230, Chapter 10 at pages 196-198).

Particular mention may be made in this regard of *Sambucus nigra* L. Lectin, *Sambucus sielbodiana* lectin wheatgerm agglutinin, *Maackia amurensis* lectin, and *E. coli* K99 lectin. *S.* nigra L. lectin is particularly effective when used on its own, although it may equally effectively be used in combination with other lectins eg. ConA.

Some particular combinations of carbohydrate binding ligands useful for performance of the present invention are lectins from *Helicobacter pylori* and *Ricinus communis*; lectins from *Ricinus communis* and *Sambuccus nigra*; lectins from *Crotalaria junctae* and *Sambuccus nigra*; lectins from *Crotalaria junctae* and *Helicobacter pylori* and lectins from *Ricinus communis* and anti-sialic acid antibodies. The most preferred of the combinations are those which incorporate galactose-binding and sialic acid-binding ligands.

Preferably, lectins which bind to the mono- and oligosaccharide arrangements of the transferrin carbohydrate side chains with a kD of 10⁴ or greater are used. Lectins with a lower binding affinity may also be used, but preferably at a higher density.

When the body fluid comprising transferrin variants is contacted with the carbohydrate-binding ligands, substantially all of the variants with carbohydrate side chains or remnants thereof are retained by the carbohydrate-binding ligands and only the carbohydrate-free transferrin is not bound to the ligands. The unbound, carbohydrate-free transferrin containing fraction (ie. the substantially carbohydrate-free fraction) may then be separated from the other variants and collected by any suitable means.

In its most general sense, the method of the invention involves simply contacting the sample with the carbohydrate-binding ligand(s) and separating a fraction which does not bind. Where more than one ligand is being used, these may be used together or they may be used individually, for example, sequentially. Following the binding step(s) a fraction may conveniently be collected which does not bind and which contains the CFT. As mentioned above, the collection may be by any suitable means, for example, precipitation, centrifugation, filtration, chromatographic methods etc. Where different carbohydrate-binding ligands are used individually, different separation/collection formats may be used for each individual binding step.

Precipitation of carbohydrate-containing moieties in the sample may be achieved using lectins having known "precipitation" properties ie. lectins capable of inducing precipitation of the moieties to which they bind. Combinations of lectins may advantageously be used for such a precipitation procedure, since differing lectin specificities increase the number of available binding sites. The non-binding ("carbohydrate-free") fraction may then readily be collected, for example by centrifugation or filtration to separate the precipitate.

In alternative embodiments, the carbohydrate binding ligand(s) may conveniently be immobilised to facilitate the separation and collection of the carbohydrate-free, non-binding fraction. It is well known in the art to immobilise carbohydrate-binding ligands such as lectins for separation purposes, for example, in chromatographic columns, and any lectin affinity chromatography method known in the art could for example be used (see for example, Cummings (1994) Methods in Enzymology **230:**66-86).

The carbohydrate-binding ligands may be immobilised by binding or coupling to any of the well known solid supports or matrices which are currently widely used or proposed for immobilisation or separation etc. These may take the form of particles, sheets, gels, filters, membranes, fibres or capillaries or microtitre strips, tubes or plates or wells etc and conveniently may be made of glass, silica, latex or a polymeric material. Techniques for binding the ligand to the solid support are also extremely well known and widely described in the literature. For example, the carbohydrate-binding ligands used may conveniently be coupled covalently to CNBr-activated Sepharose or N-hydroxysuccinimide-activated supports, optionally in the presence of low molecular weight haptens to protect the carbohydrate binding sites on the ligand. Other coupling methods for proteins are also well known in the art.

Batch separations using immobilised carbohydrate-binding ligands may be performed using a range of different formats which are known in the art.

In a different embodiment, although this is less preferred, the immobilised carbohydrate-binding ligands may be packed or arranged into a column. The body fluid comprising transferrin may be applied to the column and the transferrin variants therein contacted with the carbohydrate-binding ligands. The unbound fraction comprising CFT is separated from the bound fraction and collected.

The shape and geometry of such a column may vary depending upon the carbohydrate-binding ligands used. For example, if lectins are used as the carbohydrate-binding ligands, at low lectin concentrations a long, thin column of immobilized lectins is preferred. At high lectin concentrations, column geometry is less crucial.

Columns may be constructed using any method known in the art. If lectins are to be used as the carbohydrate-binding ligands, the columns may be constructed in either glass tubes or preferably in disposable plastic pipettes of any desired capacity. Smaller volumes may however be preferred due to economic considerations. Columns are preferably stored at around 4°C prior to use.

The column may be flushed through with an eluant to allow or facilitate collection of the unbound fraction, in which case the eluant should preferably be administered using a calibrated micropipette to ensure the correct volume is administered. The volume administered is preferably within 3% of the desired (ie. calibration) volume, more preferably, within 1 or 2%. Since the rate of binding to oligosaccharides is comparatively slow, especially with plant lectins, it is preferable that slow flow rates are employed to maximise lectin/carbohydrate interactions. The eluant will generally be at a temperature within 5°C of the desired (calibration) value, e.g. 25°C, and more preferably within 1°C.

When using combinations of carbohydrate-binding ligands in a column format, either sequential columns using different ligands may be used or different ligands may be used in the same column material, either as a mixture or in a column comprising different layers, each layer having a different ligand..

In an alternative embodiment, the carbohydrate-binding ligand may be immobilised on a particulate solid phase, for example, latex, silica or polymer beads. To aid manipulation and separation, magnetic beads may be used. The term "magnetic" as used herein means that the support is capable of having a magnetic moment imparted to it when placed in a magnetic field. In other words, a support comprising magnetic particles may readily be removed by magnetic aggregation, which provides a quick, simple and efficient way of separating the fractions following the carbohydrate binding step.

Thus, using the method of the invention, the magnetic particles with carbohydrate or carbohydrate-containing moieties attached may be removed onto a suitable surface by application of a magnetic field, for example, using a permanent magnet. It is usually sufficient to apply a magnet to the side of the vessel containing the sample mixture to aggregate the particles to the wall of the vessel and to collect the remainder of the sample, which will comprise the "non-binding, CFT-containing fraction" which may be returned for subsequent analysis.

Especially preferred are superparamagnetic particles, which include for example those described by Sintef in EP-A-106873, as magnetic aggregation and clumping of the particles during the reaction can be avoided. Magnetic particles are commercially available from a number of sources, including for example, Advanced Magnetics Inc., (USA), Amersham (UK), Bang Particles (USA), and Dynal AS (Oslo, Norway).

Functionalised coated particles for use in the present invention may be prepared by modification of the beads, for example according to US patents 4,336,173, 4,459,378 and 4,654,267. Thus, beads, or other supports, may be prepared having different types of functionalised surface, for attachment of a desired carbohydrate-binding ligand.

Separations based on centrifugation and/or filtration are convenient. In a preferred embodiment a centrifuge tube (eg. Eppendorf tube) and "filter cup" format may be used, and such formats are readily commercially available, for example from Millepore. Thus the sample and carbohydrate-binding ligand may be added to the cup in the tube and allowed to bind. The tube (and cup) is then spun, and the non-binding supernatant collects in the tube. The carbohydrate-binding ligand may be such as to induce precipitation of the bound carbohydrate moieties or it may be immobilised, for example as a slurry eg. a gel or on particles. In either case, the bound carbohydrate binding fraction is retained in the cup.

As a variation of such a "tube and cup" arrangement, the cup may be provided with one or more "discs" or filters which carry immobilised carbohydrate-binding ligands.

In an optional embodiment of the invention, an additional step of removing or depleting certain carbohydrate-carrying (glycosylated) transferrin isoforms by other separation procedures can be carried out prior to the carbohydrate-binding ligand binding step, step (a). This may be desirable, for example, for economic reasons to limit the amount of binding ligand necessary in step (a), e.g. if expensive lectins are being used. Conveniently, such removal or depletion may be achieved by ion exchange chromatography to remove e.g. all or substantially all of the hexa-, penta-, tetra- and 'tri-sialotransferrins, and preferably also some or most of the disialotransferrin component. As mentioned above, ion exchange as a means of separating the various isotransferrin components is well known, and is described for example in US-A-4626355, Schellenberg *et al*., (supra), Heil *et al*., (supra) and WO96/26444. Advantageously, an anion exchange chromatography step may be used, with the chromatography conditions (e.g. pH and ion binding strength) selected to permit retention of the desired transferrin variants (e.g. hexa-, penta-, tetra- and tri-sialo transferrin, and optionally some or all of the disialo fraction).

Appropriate conditions e.g. buffering-capacity of the resin, sample/equilibration/elution buffer pH and/or ionic strength can readily be determined according to techniques known in the art, and according to the separation desired to be achieved (ie. which and how much of the glycosylated transferrin variants it is desired to separate, which may be according to choice). As is known in the art, prior to ion exchange, the sample may be treated with iron-containing buffer to saturate the iron-binding sites in the transferrin molecules in the sample.

Conveniently, according to techniques known in the art, chloride may be used as the counterion in the ion exchange procedure in order to achieve the desired separation. Thus, appropriate amounts of chloride ion present in the chromatography procedure necessary to achieve retention of the desired transferrin variants may be determined by routine experiments, and may depend on the precise conditions, batch of chromatography medium etc. The procedure can be monitored by isoelectric forcussing or HPLC analysis, again according to standard techniques known in the art. The resolution obtainable between different glycosylated transferrin variants may depend on the exact nature of the procedure, chromatography format (ie. batch slurry form or column format etc), and this may be selected according to choice, convenience etc.

The ion exchange chromatography step may be carried out in any convenient manner known in the art according to choice e.g. in a batch or column format. Likewise, the conditions may be selected to achieve the separation (ie. depletion or removal) in any desired manner, for example by retaining the isotransferrin variants it is desired to remove, or by pre-treating the sample by ion exchange such that the "undesired" glycosylated variants do not absorb to the medium, and the remainder.of the sample is separated and then eluted from the ion exchange medium, prior to being subjected to step (a) of the method.

Advantageously however, the chromatography conditions are set to permit retention of the "undesired" glycosylated variants. Further advantageously, the ion-exchange medium may simply be added to the sample to bind the glycosylated variants, followed thereafter by addition of the carbohydrate-binding ligand to the sample; this will react first with any transferrin variant molecules in solution, before it' binds to any variants which are retained in the ion exchange medium.

As exemplary of ion exchange conditions which may be used, mention may be made of Whatman QASL anion exchange resin buffered at pH 6.3, which may be used to bind the hexa-, penta-, tetra- and trisialo transferrins and most of the disialo transferrins.

It has been found that using a simple batch format, separation of all the disialotransferrin fraction may be difficult to achieve, without separating also some of the CFT fraction. Nonetheless, an advantageous and effective assay system may still be achieved by separating only a portion of the disialofraction.

Following the binding and separation steps, the content of transferrin in the "non-binding" fraction is determined. As mentioned above, most conveniently the separated fraction comprising the CFT variant is assessed for CFT,content. Alternatively, however the transferrin contents of the initial sample, and the fraction(s) binding to the carbohydrate binding ligand may be determined, and the transferrin content of the "non-binding" fraction determined by subtraction. This may be done by any standard procedure known in the art for assay of transferrin, for example, by any standard immunoassay technique, e.g. an ELISA or radioimmunoassay technique.' Methods for determining transferrins are described for example in US-A-4,626,355 (Joustra).

Many commercial assays for transferrin are available and have been described in the literature. For example an RID (radio immuno diffusion) assay based on the method of Mancini is available from Hoechst (see Mancini et al., Immunochemistry, 2: 235-254 (1965)). A rocket immuno electrophoresis method is described by Laurell in Scand. J. Clin. Lab. Invest. 29 (Suppl. 124): 21-37 (1972). Particular mention may also be made of the particle-based immunoassay method of Müller et al., in Lab. Med., 15: 278 (1991). This is a very sensitive technique, based on an enhanced turbidometric method which uses a turbidometric signal but is more sensitive than traditional turbidometric methods.

An important advantage of the assay method of the present invention is that it enables transferrin detection methods to be used which do not require further separation steps (e.g. nephelometry) and in particular, it allows turbidimetric determination of CFT content. This is important since although nephelometry is in general a more sensitive assay for opacity in a fluid sample than is turbidimetry, the technique requires a non-linear optical path for the detection. apparatus and thus is not readily adapted for inclusion within existing multi-task light-absorbance determination based diagnostic assay apparatus, in which the light path through the sample is linear.

For either turbidimetric or nephelometric CFT determination, opacity will generally be generated by contacting the separated fraction or an aliquot thereof with an anti-transferrin antibody or antibody fragment, e.g. a rabbit anti-human transferrin antibody such as is commercially available from Dako of Copenhagen, Denmark. The' Dako antibodies are specific to transferrin and show no cross reactions with other blood proteins that may be present in the eluate. The quantity of antibody used should of course be optimised against transferrin containing standard samples as opacification arises from the hook effect whereby multiple transferrin binding generates the opacification centres.

In the case of "tube and cup" embodiment described above for example, the anti-transferrin antibodies may simply be added to the tube after centrifugation.

As in routine turbidimetric and nephelometric assays, a polymeric opacification enhancer, such as polyethyleneglycol, is preferably also added to the eluate.

In determining CFT content using such measuring techniques, a kinetic reading mode may of course be used.

Before the nephelometric or turbidimetric determination is made, the fraction, antibody and enhancer may be incubated for a short period, e.g. 5 minutes to an hour for end-point measurements, preferably about 10 minutes.

The light used in the determination of opacification should have an appropriate wavelength. In this regard we have found that use of a 405 nm filter, or more preferably a 340 nm filter, yields particularly good results.

In general, besides the sample under evaluation, calibration samples with known transferrin contents will also be assessed in the performance of the assay method of the invention. Such determinations can be used to plot a calibration curve from which the CFT content of the sample under evaluation may be determined. Preferably calibration samples having transferrin contents of up to 0.05mg/mL (e.g. 0.002, 0.01, 0.02 and 0.03mg/ml) will be used. (These will not of course be passed through the carbohydrate-binding ligands to separate out the carbohydrate-containing variants).

Moreover in the assay method of the invention the total transferrin content of the sample may preferably be determined, using the same assay procedure (i.e. turbidimetry etc). In this way the CFT content may be determined as a percentage of total transferrin (%CFT). %CFT may be a more precise marker for alcohol consumption than total CFT, and a threshold value, for example 1%, may be set. From a diagnostic point of view however, it may reasonably be assumed that the presence of any CFT whatsoever is indicative of alcohol abuse.

Alternatively, the CFT may be assessed as an actual concentration (ie. a mass per unit volume).

Viewed from a further aspect, the invention provides a kit for a diagnostic assay according to the invention, said kit comprising:
one or more carbohydrate-binding ligands; and
means for the detection of transferrin.

Conveniently, the kit may also comprise a transferrin standard or standards for reference, and preferably the means for detecting transferrin are for the turbidometric determination of transferrin. Thus, in one preferred embodiment, the kit of the invention may comprise:
preferably, a transferrin solution of known concentration and more preferably a set of such solutions having a range of transferrin concentrations;
one or more carbohydate-binding ligands, optionally immobilised on a solid support;
preferably, a light transmitting eluate receiving vessel;
preferably, an anti-transferrin antibody or antibody fragment; and preferably, an opacification enhancer.

If desired an automated apparatus may be arranged to receive a transferrin containing body fluid sample, apply the sample to a solid support carrying one or more carbohydrate-binding ligands, collect a CFT containing fraction, apply an opacifying anti-transferrin antibody or antibody fragment, and determine CFT content in the eluate. Such apparatus is also deemed to fall within the scope of the invention.

A particular advantage of the present invention over the prior art is that all samples used in ion-exchange procedures such as are described in the prior art for CDT determinations, require to be diluted to facilitate good ion-exchange separation. The samples used in the present invention do not require dilution and hence less materials are consumed and less preparatory steps are required in sample preparation.

All the methods and assays of the prior art, including those which are currently being exploited commercially, are based on the identification and quantitation of different transferrin variants on the basis of differences in charge and hence pI of the different variants. Where the primary structure ie. the amino-acid sequence of transferrin variants is constant, these differences in charge arise due to the loss of negatively charged sialic acid residues, which increases the pI of the transferrin variants incrementally with each sialic acid residue lost:

However, the primary structure of the transferrin polypeptide is known to be polymorphic and the prevalence of particular amino-acid sequence isoforms differs according to racial origin. For example, relative to "normal" transferrin which predominates in caucasian populations, the transferrin D variant possesses a single, non-conservative amino-acid substitution in the polypeptide backbone which affects the isoelectric point of the transferrin variant. The D variant is common within populations of Japanese and black African origin. The non-conservative amino-acid substitution changes the net charge and hence pI of the transferrin backbone with the result that in isoelectric focussing or equivalent studies, many false positive results are'generated in relation to persons of Japanese or black African origin. Clearly this is unacceptable, and means that in populations where the transferrin D variant is common, a second test must be carried out to establish which transferrin variant is expressed by the individual under study. This adds greatly to the overall cost, time taken and complexity of the assessment of alcoholism.

The assay of the present invention relies solely on the presence or absence of carbohydrate moieties associated with the polypeptide backbone of transferrin. Since it is not influenced by polymorphisms in the amino acid sequence, it is not subject to any false positives or negatives on account of the variant polymorphism expressed by the individual under clinical evaluation. Hence, the present invention is particularly advantageous in that it is racially independent.

The invention will now be illustrated by the following non-limiting Examples and the accompanying figures in which:
Figure 1 shows the glycosylated transferrin binding capacity of a column comprising immobilized Con A and SNA lectins. Fraction 1 contains the CFT;
Figure 2 shows the elution profile of an SNA lectin column of neuraminidase treated transferrin (removes sialic acid residues) (filled circles) and serum from a non-alcoholic (open circles). The fraction between 1.5 ml and 2.5 ml contains the transferrin lacking sialic acid residues.

### Example 1

### Quantitation of CFT by means of immobilised lectin from Sambuccus Nigra.

a. 10 µl serum samples are mixed with 0.5 ml 20 mM TRIS buffer pH = 7.5 comprising 150 mM sodium chloride.
b. 0.5 ml agarose elderberry bark (*Sambuccus Nigra*) lectin (supplied by Vector Laboratories, Burlingame, USA) is suspended in 0.5 ml 20 mM TRIS buffer pH = 7.5 comprising 150 mM sodium chloride, and then mixed with each of the serum samples in TRIS buffer (see a. above).
c. The suspensions are transferred to Ultra-free MC Millipore UFC3 OHV (0.45 µm) filter cups and centrifuged.
d. Mix 200 µl of the filtrate with 200 µl of an anti-transferrin antibody solution comprising 0.27 M TRIS, 4.5% PEG 8000, 4.3 mM sodium azide, 1:10 dilution of Dako anti-human-transferrin antibodies Q0327, and HCl to pH = 7.4.
e. Read the turbidimetric/nephelometric signal.

### Example 2

### Quantification of CFT by means of surface lectin on Helicobacter pylori.

a. Helicobacter *pylori* is cultivated and isolated according to Lelwala *et al*., "Isolation of a sialic acid-specific surface haemaglutinin of *Helicobacter* *pylori* strain NCTC11637", Zblatt 280:93-196, 1993.
b. 25 µl serum sample is mixed with 0.5 ml 20 mM Tris-HCl buffer with pH = 7.5 containing 150 mM sodium chloride.
c. A suspension of Helicobacter pylori with a binding capacity in excess of the total glycoprotein concentration of the serum sample is added.
d. The suspensions are transferred to Ultra-free MC Millipore UFC3 OHV (0.45 µm) filter cups and centrifuged.
e. Mix 200 µl of the filtrate with 200 µl of a anti-transferrin antibody solution comprising 0.27 M TRIS, 4.5% PEG 8000, 4.3 mM sodium azide, 1:10 dilution of Dako anti-human-transferrin antibodies Q0327, and HCl to pH = 7.4.

### Example 3

### Quantitation of CFT by means of immobilised lectin from Helicobacter pylori.

a. 10 µl serum samples are mixed with 0.5 ml 50 mM TRIS buffer pH = 7.5 comprising 150 mM sodium chloride.
b. 0.5 ml of agarose (Reacti-Gel from Pierce Chemical Company, US) with lectin from *Helicobacter communis* isolated according to Lelwala *et al*., "Isolation of a sialic acid-specific surface haemaglutinin of *Helicobacter pylori* strain NCTC11637", Zblatt 280:93-196, 1993 immobilised on the agarose according to the manufacturer of the "Recti-Gel" package insert, suspended in a buffer of 20 mM TRIS buffer pH = 7.5 comprising 150 mM sodium chloride, and then mixed with each of the serum samples in TRIS buffer (see a. above).
c. The suspensions are transferred to Ultra-free MC Millipore UFC3 OHV (0.45 µm) filter cups and centrifuged.
d. Mix 200 µl of the filtrate with 200 µl of an anti-transferrin antibody solution comprising 0.27 M TRIS, 4.5% PEG 8000, 4.3 mM sodium azide, 1:10 dilution of Dako anti-human-transferrin antibodies Q0327, and HCl to pH = 7.4.
e. Read the turbidimetric/nephelometric signal.

### Example 4

### Quantitation of CFT by means of immobilised lectin from Sambuccus nigra in a column format.

a. 10 µl serum samples are mixed with 0.5 ml binding buffer 20 mM Tris-HCl buffer with pH = 7.5 containing 150 mM sodium chloride.
b. Each diluted serum samples are passed through a column of 0.5 ml agarose elderberry bark (*Sambuccus nigra*) lectin (supplied by Vector Laboratories, Burlingame, USA) is suspended in 20 mM Tris-HCl buffer with pH = 7.5 containing 150 mM sodium chloride, and another 1.0 ml of the same buffer is passed through the column.
c. Mix 200 µl of the eluted solution with 200 µl of an anti-transferrin antibody solution comprising 0.27 M TRIS, 4.5% PEG 8000, 4.3 mM sodium azide, 1:10 dilution of Dako anti-human-transferrin antibodies Q0327, and HCl to pH = 7.4.
d. Read the turbidimetric/nephelometric signal.

### Example 5

### Quantitation of CFT by means of immobilised lectin from Ricinus communis and Sambuccus nigra.

a. 10 µl serum samples are mixed with 0.5 ml binding buffer 0.5 ml 20 mM TRIS buffer pH = 7.5 comprising 150 mM sodium chloride.
b. 0.5 ml agarose elderberry bark (*Sambuccus nigra*) lectin (suppled by Vector Laboratories, Burlingame, USA) and 0.5 ml agarose bound *Ricinus communis* lectin from EY Laboratories (US) was mixed with 0.5 ml 20 mM TRIS buffer pH = 7.5 comprising 150 mM sodium chloride and then mixed with the diluted serum samples from (a). The use of both galactose-binding lectin and sialic acid binding lectin in combination ensures a good binding of all carbohydrate-containing transferrin molecules.
c. The suspensions are transferred to Ultra-free MC Millipore UFC3 OHV (0.45 µm) filter cups and centrifuged.
d. Mix 200 µl of the filtrate with 200 µl of an anti-transferrin antibody solution comprising 0.27 M TRIS, 4.5% PEG 8000, 4.3 mM sodium azide, 1:10 dilution of Dako anti-human-transferrin antibodies Q0327, and HCl to pH = 7.4.
e. Read the turbidimetric/nephelometric signal.

### Example 6

### Anion exchange pre-treatment step

20 µl of serum sample is mixed with 0.5 ml of a 25% preswollen Whatman QA52 anion exchange resin suspended in 20mM Bis(2-hydroxy)amino-tris(hydroxymethyl)methane pH = 6.3. The chloride content of the medium is carefully adjusted to separate the desired isotransferrin fractions, and this may be monitored by HPLC or isoelectric focussing. Thereafter, 0.5ml agarose elderberry bark lectin (Vector Laboratories) is added, and the suspension is mixed gently. The suspension is thereafter filtered by centrifugation in a Millipore Ultra-Free MC UFC3 OHV filter cup, and the filtrate is collected. 200 µl of the filtrate is mixed with 200 µl of an transferrin antibody (Dako) solution diluted 1:10 in 0.27M TRIS, 4.5% PEG 8000, 4.3 mM sodium azide pH = 7.4, and the nephelometric signal is read and interpolated in a standard curve constructed from standards of known concentration of human transferrin.

### Example 7

### Quantitation of CFT by means of immobilised lectins

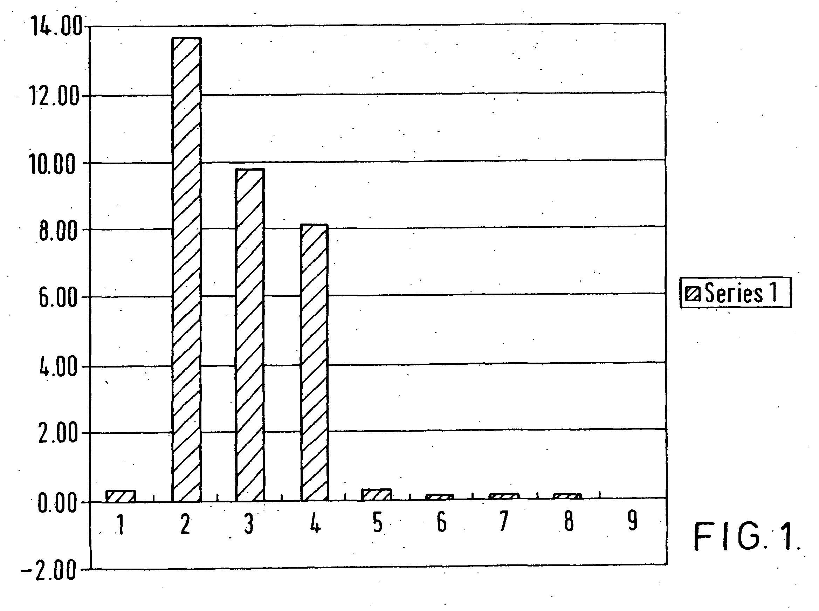

a. A 10 µl serum sample from an alcoholic was mixed with 0.5 ml binding buffer (25 mM TRIS, 1 mM CaCl₂, 1 mM MnCl₂, buffer pH = 7.5 comprising 150 mM sodium chloride).
b. 0.5 ml immobilised lectin mixture comprising 400 µl sepharose ConA lectin (Pharmacia Sweden) and 100 µl agarose elderberry bark (*Sambuccus nigra*) lectin (suppled by Vector Laboratories, Burlingame, USA) here suspended in the sample/binding buffer mixture (see a. above).
c. The suspension was transferred to Ultra-free MC Millipore UFC3 OHV (0.45 m) filter cups and centrifuged.
d. 200 µl of the filtrate (Fraction 1) was mixed with 100 µl of an anti-transferrin antibody solution comprising 0.27 M TRIS, 4.5% PEG 8000, 4.3 mM sodium azide, 1:10 dilution of Dako anti-human-transferrin antibodies Q0327, and HCl to pH = 7.4.
e. 500 µl elution buffer (50 mM Tris Buffered Saline + 0.25 M lactose which causes elution from SNA and 200 mM α-D-methyl-d glycoside which causes elution from Con A) was added to the filter cup (containing the lectins) and centrifuged. 200 µl of this filtrate (fraction 2) was mixed with 100 µl of anti-transferrin antibody solution as described in step d. This step was repeated to generated a total of 9 fractions in order to test the binding characteristics for transferrin containing glycan chains.
f. The turbidimetric signals were read for each fraction using a Turbidmetric Immuno Assay (TIA) method. The results are shown in Fig. 1 attached hereto.

### Example 8

### Quantitation of CFT by means of immobilised lectin from Sambuccus nigra in a column format - Preliminary demonstration of principle of assay

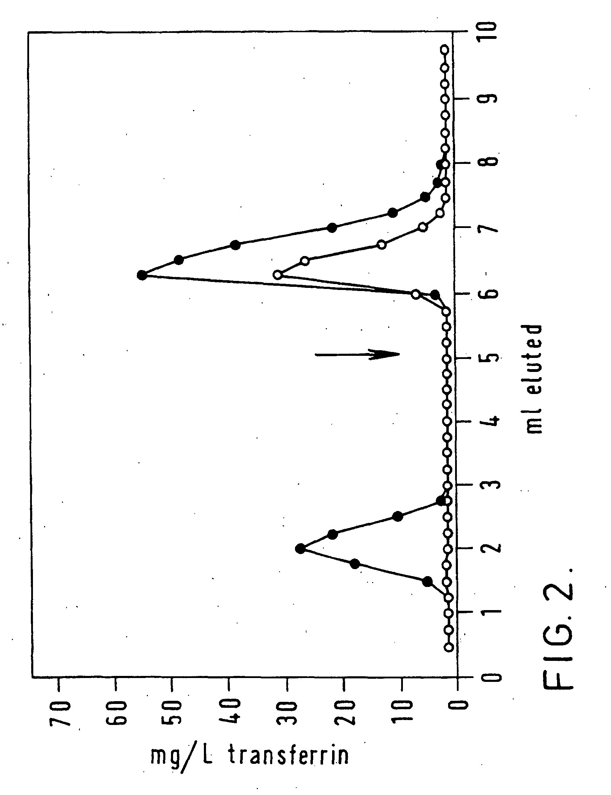

a. 50 µl serum sample from a non-alcoholic or a sample in which the transferrin has been enzymatically treated with neuraminidase (which cleaves terminal sialic acid residues from the glycan chains thus generating transferrin molecules which simulate those of an alcoholic, for the purposes of this experiment), was mixed with 0.5 ml binding buffer (50 mM TRIS-HCl buffer with pH = 7.5 comprising 150 mM sodium chloride).
b. Each diluted serum sample was passed through a column of 1.0 ml agarose elderberry bark *(Sambuccus nigra*) lectin (suppled by Vector Laboratories, Burlingame, USA) suspended in 50 mM Tris-HCl buffer with pH = 7.5 containing 150 mM sodium chloride. Another 1.0 ml of the same buffer was passed through the column. Liquid collected from the column at this point was discarded.
c. Further binding buffer was added and liquid passing through the column collected in 0.25 ml volumes. After 18 x 0.25 ml fractions were collected, an elution buffer (50 mM Tris Buffered Saline +0.25 M lactose) was added to the column to release the transferrin molecules bound to the SNA lectins.
d. 200 µl of each collected fraction was mixed with 100 µl of an anti-transferrin antibody solution comprising 0.27 M TRIS, 4.5% PEG 8000, 4.3 mM sodium azide, 1:10 dilution of Dako anti-human-transferrin antibodies Q0327, and HCl to pH = 7.4.
e. The turbidimetric signal was read from each fraction using a Turbidimetric Immuno Assay (TIA) method an the results of this Example are shown in Figure 2 attached hereto'. The arrow shows where the elution buffer was added for elution of adsorbed transferrin (containing glycan chains with sialic acid residues). The fraction between 1.5 ml and 2.5 ml contains the transferrin lacking sialic acid residues.

## Claims

1. A method for the determination of carbohydrate-free transferrin in a body fluid for use in the assessment of alcohol consumption, said method comprising
(a) contacting a sample of said body fluid with a carbohydrate-binding ligand, to bind any carbohydrate or carbohydrate-containing moieties in said sample to said ligand;
(b) separating a fraction not binding to said ligand and
(c) determining the content of transferrin in said fraction.

2. A method as claimed in claim 1, wherein the sample is blood or a blood-derived sample.

3. A method as claimed in claim 1 or claim 2, wherein the carbohydrate binding ligand is selected from antibodies or fragments thereof, lectins and mammalian or microbial carbohydrate-binding proteins, or mixtures thereof.

4. A method as claimed in any one of claims 1 to 3 wherein in step (a) a panel of different lectins are used.

5. A method as claimed in any one of claims 1 to 4, wherein the carbohydrate binding ligand is selected from Sambucus *nigra* lectin, *Sambucus sielbodiana* lectin, wheatgerm agglutinin, *Maackia amurensis* lectin, *E. coli* K99 lectin, *Helicobacter pylori* lectin, *Ricinus communis* lectin, and *Crotalaria junctae* lectin, and anti-sialic acid antibodies, and mixtures thereof.

6. A method as claimed in any one of claims 1 to 5, wherein the separation step (b) is by precipitation, centrifugation, filtration or chromatographic methods.

7. A method as claimed in any one of claims 1 to 6, wherein the carbohydrate binding ligand is immobilised.

8. A method as claimed in any one of claims 1 to 7, wherein an ion exchange step to remove or deplete carbohydrate-carrying transferrins in the sample is performed prior to step (a) .

9. A method as claimed in any one of claims 1 to 8, wherein the determination of transferrin content in step (c) is achieved by turbidometric or nephelometric means.

10. A kit for use in a method as defined in any one of claims 1 to 9, said kit comprising:
one or more carbohydrate-binding ligands; and
means for the detection of transferrin.

11. A kit as claimed in claim 10, wherein said means for detection of transferrin comprise an anti-transferrin antibody or antibody fragment; and preferably, an opacification enhancer.

12. A kit as claimed in claim 10 or claim 11, further comprising a transferrin solution of known concentration or a set of such solutions having a range of transferrin concentrations.

## Patentansprüche

1. Verfahren zur Bestimmung von Kohlenhydrat-freien Transferrin in einer Körperflüssigkeit zur Verwendung bei der Beurteilung von Alkoholkonsum, wobei man bei diesem Verfahren
a) eine Probe der Körperflüssigkeit mit einem Kohlenhydrat-bindenden Liganden in Kontakt bringt, um jegliches Kohlenhydrat oder Kohlenhydratenthaltende Gruppen in der Probe an die Liganden zu binden;
b) eine an den Liganden nicht bindende Fraktion abtrennt; und
c) den Transferringehalt dieser Fraktion bestimmt.

2. Verfahren nach Anspruch 1, wobei die Probe Blut oder eine vom Blut abgeleitete Probe ist.

3. Verfahren nach Anspruch 1 oder Anspruch 2, wobei der Kohlenhydrat-bindende Ligand ausgewählt ist unter Antikörpern oder Fragmenten davon, Lektinen und Kohlenhydrat-bindendenen Proteinen aus Säugern oder Mikroben, oder Mischungen davon.

4. Verfahren nach einem der Ansprüche 1 bis 3, wobei man in Schritt (a) einen Satz verschiedener Lektine verwendet.

5. Verfahren nach einem der Ansprüche 1 bis 4, wobei der Kohlenhydrat-bindende Ligand ausgewählt ist unter *Sambucus nigra* Lektin, *Sambucus sielbodiana* Lektin, Weizenkeim- Agglutinin, *Maackia amurensis* Lektin, *E. coli* K99 Lektin, *Helicobacter pylori* Lektin, *Ricinus communis* Lektin, und *Crotalaria junctae* Lektin, und Anti-Sialinsäure-Antikörpern und Mischungen davon.

6. Verfahren nach einem der Ansprüche 1 bis 5, wobei der Trennschritt (b) durch Fällung, Zentrifugation, Filtration oder chromatographische Methoden erfolgt.

7. Verfahren nach einem der Ansprüche 1 bis 6, wobei der Kohlenhydrat-bindende Ligand immobilisiert ist.

8. Verfahren nach einem der Ansprüche 1 bis 7, wobei ein Ionenaustausch-Schritt zur Entfernung oder Abreicherung von Kohlenhydrat-tragenden Transferrinen in der Probe vor Schritt (a) durchgeführt wird.

9. Verfahren nach einem der Ansprüche 1 bis 8, wobei die Bestimmung des Transferringehalts in Schritt (c) mit turbidimetrischen oder nephelometrischen Mitteln erfolgt.

10. Kit zur Verwendung in einem Verfahren gemäß der Definition in einem der Ansprüche 1 bis 9, wobei der Kit umfasst:
- einen oder mehrere Kohlenhydrat-bindende Liganden, und
- Mittel zur Detektion von Transferrin.

11. Kit nach Anspruch 10, wobei die Mittel zur Detektion von Transferrin einen Anti-Transferrin-Antikörper oder Antikörperfragment davon; und, vorzugsweise, einen Trübungsverstärker umfassen.

12. Kit nach Anspruch 10 oder Anspruch 11, weiter umfassend eine Transferrinlösung bekannter Konzentration oder einen Satz solcher Lösungen mit einem Transferrin-Konzentrationsbereich.

## Revendications

1. Procédé pour la détermination de transferrine exempte d'hydrates de carbone dans un fluide corporel pour utilisation dans l'évaluation de consommation d'alcool, ledit procédé comprenant les étapes consistant à :
(a) mettre en contact un prélèvement dudit fluide corporel avec un ligand liant les hydrates de carbone, pour lier tout hydrate de carbone ou des fragments contenant des hydrates de carbone dans ledit prélèvement audit ligand ;
(b) séparer une fraction non liée audit ligand et
(c) déterminer la teneur en transferrine dans ladite fraction.

2. Procédé selon la revendication 1, dans lequel le prélèvement est un prélèvement de sang ou dérivé du sang.

3. Procédé selon la revendication 1 ou la revendication 2, dans lequel le ligand liant les hydrates de carbone est choisi parmi des anticorps ou des fragments de ceux-ci, des lectines et des protéines mammifères ou microbiennes qui lient les hydrates de carbone, ou des mélanges de ceux-ci.

4. Procédé selon l'une des revendications 1 à 3, dans lequel dans l'étape (a) un éventail de lectines différentes est utilisé.

5. Procédé selon l'une des revendications 1 à 4, dans lequel le ligand liant les hydrates de carbone est choisi parmi la lectine *Sambucus nigra*, la lectine *Sambucus sielbodiana*, l'agglutinine de germe de blé, la lectine *Maackia amurensis*, la lectine K99 *E. coli*, la lectine *Helicobacter pylori*, la lectine *Ricinus communis*, et la lectine *Crotalaria junctae* et des anticorps contre l'acide sialique, et des mélanges de ceux-ci.

6. Procédé selon l'une des revendications 1 à 5, dans lequel l'étape de séparation (b) se fait par précipitation, centrifugation, filtration ou des procédés chromatographiques.

7. Procédé selon l'une des revendications 1 à 6, dans lequel le ligand liant les hydrates de carbone est immobilisé.

8. Procédé selon l'une des revendications 1 à 7, dans lequel une étape d'échange d'ions pour extraire ou réduire les transferrines porteuses d'hydrates de carbone dans le prélèvement est réalisée avant l'étape (a).

9. Procédé selon l'une des revendications 1 à 8, dans lequel la détermination de la teneur en transferrine dans l'étape (c) est obtenue par des moyens turbidimétriques ou néphélométriques.

10. Kit pour utilisation dans un procédé selon l'une des revendications 1 à 9, ledit kit comprenant :
un ou plusieurs ligands liant les hydrates de carbone ; et des moyens pour la détection de transferrine.

11. Kit selon la revendication 10, dans lequel lesdits moyens pour la détection de transferrine comprennent un anticorps ou un fragment d'anticorps contre la transferrine ; et de préférence, un amplificateur d'opacification.

12. Kit selon la revendication 10 ou la revendication 11, comprenant en outre une solution de transferrine de concentration connue ou un ensemble de telles solutions ayant une plage de concentrations de transferrine.
